# EUROPÄISCHE PATENTSCHRIFT

(11) **EP 2 041 049 B1**
(45) Veröffentlichungstag und Bekanntmachung des Hinweises auf die Patenterteilung: **25.04.2012**
(21) Anmeldenummer: 07729028.6
(22) Anmeldetag: 11.05.2007
(51) Int. Cl.: C07C 1/24, C07C 11/10

(54) **VERFAHREN ZUR HERSTELLUNG VON 3-METHYLBUT-1-EN**
PROCESS FOR PREPARING 3-METHYLBUT-1-ENE
PROCEDE DE FABRICATION DE 3-METHYLBUT-1-ENE

(30) Priorität: 11.07.2006 DE 102006031964
(43) Veröffentlichungstag der Anmeldung: 01.04.2009
(73) Patentinhaber: Evonik Oxeno GmbH, 45772 Marl (DE)
(72) Erfinder: KAIZIK, Alfred, 45772 Marl (DE); WIESE, Klaus-Diether, 45721 Haltern am See (DE); MASCHMEYER, Dietrich, 45657 Recklinghausen (DE); HESS, Dieter, 45770 Marl (DE); BÜSCHKEN, Wilfried, 45721 Haltern am See (DE); NIERLICH, Franz, 45768 Marl (DE)
(86) Internationale Anmeldenummer: PCT/EP2007/054576
(87) Internationale Veröffentlichungsnummer: WO 2008/006633

(56) Entgegenhaltungen:
- EP-A1- 0 623 383
- EP-B1- 0 369 213
- WO-A-2005/028404
- WO-A-2005/080302
- JP-A- 3 028 213
- JP-A- 3 068 613
- RU-C1- 2 032 649
- US-A1- 2004 122 278
- M MARCHETTI ET AL: "A protein-rhodium complex as an efficient catalyst for two-phase olefin hydroformylation" TETRAHEDRON LETTERS, Bd. 41, Nr. 19, 2000, Seiten 3717-3720, XP002449558
- M. LECONTE ET AL: "Stoichiometric and Catalytic Homologation of Olefins on the fischer-Tropsch Catalysts Fe/SiO2, Ru/SiO2, Os/SiO2 and Rh/SiO2. Mechanistic Implication in the Mode of C-C Bond Formation" J. AM. CHEM. SOC., Bd. 106, Nr. 4, 1984, Seiten 1141-1143, XP002449392

## Beschreibung

Die vorliegende Erfindung betrifft die Herstellung von 3-Methylbut-1-en durch eine zumindest dreistufige Synthese aus einem Isobuten aufweisenden Kohlenwasserstoffstrom.

C₅-Olefine, insbesondere Methylbutene, sind in der Industrie begehrte Einsatzstoffe. Besonders 2-Methyl-but-1-en ist ein in der Parfümindustrie und zur Herstellung von Isopren häufig verwendeter Einsatzstoff. 3-Methyl-but-1-en kann theoretisch als Monomer oder Comonomer zur Herstellung von Polymeren bzw. Copolymeren genutzt werden. Praktisch sind industriell hergestellte Copolymere von 3-Methyl-but-1-en nicht bekannt, da 3-Methyl-but-1-en in großen Mengen nicht verfügbar ist. Prinzipiell ist 3-Methyl-but-1-en zwar in C₅-Fraktionen, wie z. B. Leichtbenzin, enthalten. Der Gehalt an 3-Methyl-but-1-en in solchen Fraktionen beträgt allerdings nur von ca. 1 bis 5 Massen-%. Zudem ist die Isolierung von 3-Methyl-but-1-en aus solchen Fraktionen relativ aufwändig.

Im Stand der Technik sind einige Verfahren zur Herstellung von 3-Methyl-but-1-en beschrieben. Methylbutene können industriell z. B. durch Metathesereaktionen hergestellt werden. So beschreibt DE 199 32 060 die Herstellung von Pentenen und Methylbutenen ausgehend von einem C₄-Olefine aufweisenden Kohlenwasserstoffstrom.

In zahlreichen japanischen Anmeldungen der Mitsubishi Chemical Industry, so z. B. in JP 62-108827 wird die Herstellung von 3-Methylbut-1-en durch partielle Hydrierung von Isopren beschrieben.

In US 6,570,033 werden Bisphosphit-Metallkomplexe beschrieben, die zur Hydroformylierung einer großen Anzahl von Olefinen eingesetzt werden können. Unter anderem wird auch beschrieben, das Isobuten hydroformyliert werden kann. Die Herstellung von 3-Methylbut-1-en wird nicht erwähnt.

In WO 2005/080302 wird die Herstellung von Olefinen mit 8 bis 12 Kohlenstoffatomen aus Olefinen mit 4 bis 6 Kohlenstoffatomen durch Metathese beschrieben. Bei diesem Verfahren wird zunächst ein C₄-Kohlenwasserstoffstrom hydroformyliert, die Hydroformylierungsprodukte hydriert und aus den erhaltenen Alkoholen Wasser abgespalten. Bei der im Beispiel beschriebenen Hydroformylierung von Raffinat I, welches 28,10 Massen-% 1-Buten und 45,54 Massen-% Isobuten aufweist, wird ein Produktgemisch erhalten, welches nur 3 Massen-% 3-Methylbutanal aufweist. Auf die Hydrierung zum 3-Methylbutan-1-ol wurde verzichtet und zur Herstellung von 3-Methylbut-1-en aus 3-Methylbutan-1-ol wurde stattdessen eine käufliches 3-Methylbutan-1-ol eingesetzt.
WO2005/080302 offenbart ein Verfahren, bei welchem ein Isobuten aufweisender Kohlenwasserstoffstrom einer Hydroformylierung zugeführt wird und in der Isobuten in Gegenwart eines Rhodium-Katalysators zu einem Aldehyd hydroformyliert wird. WO2005/08030 ist auf die Herstellung von längerkettigen Olefinen durch Metathese unter Ethylenabspaltung ausgerichtet, nicht aber auf die Herstellung von 3-Methylbut-1-en. Das Ziel von WO2005/08030 ist also nicht die Hydroformylierung von Isobuten-reichen Gemischen zu 3-Methylbutanal, sondern die Herstellung von n-Pentanal. Auf die Hydrierung zum 3-Methylbutan-1-ol wird in D1 verzichtet und zur Herstellung von 3-Methylbut-1-en aus 3-Methylbutan-1-ol wurde stattdessen ein käufliches 3-Methylbutan-1-ol eingesetzt, um das daraus erhaltene 3-Methylbut-1-en in der Metathese einzusetzen.

Marchetti et al. [M Marchetti et al: A protein-rhodium complex as an efficient catalyst for two-phase olefin hydroformylation. Tetrahedron Lett. 41 (2000), 311 7-3720] und W02005/02840 zeigen, dass die Hydroformylierung von Isobuten möglich ist.

Mit keinem der im Stand der Technik beschriebenen Verfahren ist es auf einfache Weise und mit befriedigenden Umsätzen möglich, aus üblichen technischen C₄-Kohlenwasserstoff-haltigen Strömen 3-Methylbut-1-en herzustellen.

Es war deshalb Aufgabe der vorliegenden Erfindung, ein einfaches und wirtschaftliches Verfahren zur Herstellung von 3-Methylbut-1-en aus technisch in großen Mengen zur Verfügung stehenden C₄-Kohlenwasserstoff-haltigen Strömen bereitzustellen, welches einen oder mehrere der Nachteile der im Stand der Technik beschriebenen Verfahren vermeidet.

Überraschenderweise wurde nun gefunden, dass 3-Methybut-1-en auf besonders einfache Weise aus technischen Kohlenwasserstoffströmen hergestellt werden kann, indem ein Kohlenwasserstoffstrom, der zumindest 70 Massen-% Isobuten in Bezug auf die im Kohlenwasserstoffstrom enthaltenen Olefine aufweist, einer Hydroformylierung zugeführt wird, in der Isobuten in Gegenwart eines Rhodium-Katalysators hydroformyliert wird, das erhaltene 3-Methylbutanal zu 3-Methylbutan-1-ol hydriert wird und aus dem 3-Methylbutan-1-ol Wasser abgespalten wird.

Gegenstand der vorliegenden Erfindung ist demnach ein Verfahren zur Herstellung von 3-Methylbut-1-en aus einem Isobuten aufweisenden Kohlenwasserstoffstrom I, welches dadurch gekennzeichnet, dass
a) ein Kohlenwasserstoffstrom II, der zumindest 70 Massen-% Isobuten in Bezug auf die im Kohlenwasserstoffstrom enthaltenen Olefine aufweist und der aus dem Kohlenwasserstoffstrom I erhalten wurde oder mit diesem identisch ist, einem Verfahrensschritt der Hydroformylierung zugeführt wird, in der Isobuten in Gegenwart eines Rhodium-Katalysators hydroformyliert wird,
b) der in Schritt a) aus der Hydroformylierung von Isobuten erhaltene Aldehyd zu dem entsprechenden Alkohol hydriert wird und
c) aus dem in Verfahrensschritt b) erhaltenen zumindest einem Alkohol durch Wasserabspaltung 3-Methylbut-1-en hergestellt wird.

Der Vorteil des erfindungsgemäßen Verfahren liegt darin, dass aus großtechnisch in großen Mengen zur Verfügung stehenden C₄-Kohlenwasserstoffströmen auf einfache Weise 3-Methylbut-1-en hergestellt werden kann. Enthalten die eingesetzten Kohlenwasserstoffströme eine geeignet hohe Konzentration an Isobuten können diese Kohlenwasserstoffströme direkt der Hydroformylierung zugeführt werden. Werden Kohlenwasserstoffströme eingesetzt, die eine Konzentration von kleiner 70 Massen-% Isobuten in Bezug auf die im Strom enthaltenen Olefine aufweisen, können aus diesen auf einfache Weise durch großtechnisch erprobte Verfahren Kohlenwasserstoffströme mit geeigneter Konzentration erhalten werden.

Werden in dem erfindungsgemäßen Verfahren Kohlenwasserstoffströme eingesetzt, die eine hohe Konzentration an Isobuten aufweisen, so kann auf eine aufwändige Nachbehandlung des in Verfahrensschritt c) erhaltenen 3-Methylbut-1-en verzichtet werden. Dies ist insbesondere dann der Fall, wenn reines Isobuten, oder ein Kohlenwasserstoffstrom, der mehr als 99 Massen-% Isobuten bezogen auf die enthaltenen C₄-Olefine aufweist, eingesetzt wird.

Das erfindungsgemäß hergestellte 3-Methylbut-1-en bzw. ein erfindungsgemäß hergestelltes 3-Methylbut-1-en aufweisendes Gemisch hat außerdem den Vorteil, dass es direkt, insbesondere ohne aufwändige Reinigungsprozesse in einem Metallocen-katalysierten Copolymerisationsverfahren eingesetzt werden kann, in welchem 3-Methylbut-1-en mit Ethen oder Propen, insbesondere mit Ethen copolymerisiert wird. Dies ist überraschend, da bei der direkten Verwendung von kommerziell erhältlichem 3-Methylbut-1-en häufig eine starke Aktivitätsabnahme des Katalysators zu beobachten ist.

Die Erfindung macht sich die an sich bekannten Verfahren der Hydroformylierung, Hydrierung und Wasserabspaltung von Olefinen zunutze. Durch die erfindungsgemäße Verkettung der einzelnen Reaktionsschritte zu einem Verfahren zur Herstellung von 3-Methylbut-1-en werden die genannten Vorteile erzielt.

Der erfindungsgemäße Verfahren und die mit ihm hergestellten Produkte werden nachfolgend beispielhaft beschrieben.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden nachfolgend Schriften zitiert, so soll deren Inhalt vollständig zum Offenbarungsgehalt der vorliegenden Schrift gehören.

Das erfindungsgemäße Verfahren zur Herstellung von 3-Methylbut-1-en aus einem Isobuten aufweisenden Kohlenwasserstoffstrom I, zeichnet sich dadurch aus, dass
a) ein Kohlenwasserstoffstrom II, der zumindest 70 Massen-% Isobuten in Bezug auf die im Kohlenwasserstoffstrom enthaltenen Olefine aufweist und der aus dem Kohlenwasserstoffstrom I erhalten wurde oder mit diesem identisch ist, einem Verfahrensschritt der Hydroformylierung zugeführt wird, in der Isobuten in Gegenwart eines Rhodium-Katalysators hydroformyliert wird,
b) der in Schritt a) aus der Hydroformylierung von Isobuten erhaltene Aldehyd zu dem entsprechenden Alkohol hydriert wird und
c) aus dem in Verfahrensschritt b) erhaltenen zumindest einem Alkohol durch Wasserabspaltung 3-Methylbut-1-en hergestellt wird.

Vorzugsweise wird in Verfahrensschritt a) ein Kohlenwasserstoffstrom II eingesetzt, der zumindest 85 Massen-%, bevorzugt 95 Massen-% und besonders bevorzugt 98 Massen-% Isobuten bezogen auf die enthaltenen Olefine aufweist. Es kann besonders vorteilhaft sein, wenn ein Kohlenwasserstoffstrom II eingesetzt wird, der zumindest 85 Massen-%, bevorzugt 95 Massen-%, besonders bevorzugt 98 Massen-% und ganz besonders bevorzugt 99 Massen-% Isobuten bezogen auf die Gesamtmasse des Kohlenwasserstoffstroms aufweist. Ganz besonders vorteilhaft kann es sein, als Kohlenwasserstoffstrom reines Isobuten zu verwenden. Durch die Verwendung eines Kohlenwasserstoffstroms II, der einen möglichst hohen Anteil an Isobuten aufweist, kann in Verfahrensschritt c) ein 3-Methybut-1- aufweisender Strom erhalten werden, der nur wenige Nebenprodukte/Verunreinigungen enthält und der direkt als Edukt einer Polymerisation/Copolymerisation zugeführt werden kann.

Die Isobuten aufweisenden Kohlenwasserstoffströme I oder II, die im erfindungsgemäßen Verfahren eingesetzt werden können, können aus den unterschiedlichsten Quellen stammen. Die bedeutendste Quelle für C₄-Olefine ist der C₄-Schnitt des Crackbenzins aus Steamcrackern. Daraus wird nach Extraktion des Butadiens (z. B. durch Extraktionsdestillation) oder dessen Selektivhydrierung zu einem n-Butengemisch ein Kohlenwasserstoffgemisch (Raffinat I oder hydriertes Crack-C₄) hergestellt, das üblicherweise Isobuten, 1-Buten und die beiden 2-Butene enthält. Ein anderer Rohstoff für C₄-Olefine ist der C₄-Schnitt aus FCC-Anlagen. C₄-Olefine, hergestellt durch Fischer-Tropsch-Synthese, sind nach Selektivhydrierung des darin enthaltenden Butadiens zu n-Butenen ebenfalls ein geeigneter Einsatzstoff. Darüber hinaus können Olefingemische, die durch Dehydrierung von C₄-Kohlenwasserstoffen oder durch Metathesereaktionen erhalten werden, oder andere technische Olefinströme geeignete Einsatzstoffe sein.

Als Kohlenwasserstoffstrom I oder II kann insbesondere ein Gemisch eingesetzt werden, welches Olefine mit 3 bis 5 Kohlenstoffatomen aufweist. Bevorzugt werden als Kohlenwasserstoffstrom I oder II, insbesondere als Kohlenwasserstoffstrom II solche Gemische eingesetzt, die ausschließlich C₄-Kohlenwasserstoffe aufweisen. Als Kohlenwasserstoffstrom I oder II kann insbesondere ein Gemisch eingesetzt wird, das Isobuten und lineare Butene aufweist oder aus diesen besteht. Als Kohlenwasserstoffstrom I oder II, insbesondere als Kohlenwasserstoffstrom I, kann bevorzugt ein C₄-Schnitt, ausgewählt aus Raffinat I, selektiv hydriertem Crack-C₄, C₄-Schnitten aus FCC-Anlagen oder C₄-Olefinen, hergestellt durch Fischer-Tropsch-Synthese, eingesetzt werden. Als Kohlenwasserstoffstrom I werden bevorzugt solche technische C₄-Schnitte eingesetzt, die einen Isobutengehalt von größer 3 Massen-%, vorzugsweise größer 10 Massen-% und besonders bevorzugt größer 20 Massen-% aufweisen.

Häufig weist der Kohlenwasserstoffstrom I, der im erfindungsgemäßen Verfahren eingesetzt werden soll, einen Isobutengehalt auf, der nicht der Spezifikation für den Kohlenwasserstoffstrom II entspricht. In diesem Fall wird vor dem Verfahrensschritt a) vorzugsweise eine Verfahrensstufe 1) durchgeführt, in der aus dem Isobuten aufweisenden Kohlenwasserstoffstrom I ein Kohlenwasserstoffstrom II mit einer höheren Konzentration an Isobuten erhalten wird, der dem Verfahrensschritt a) zugeführt wird.

Weist der Kohlenwasserstoffstrom I neben Olefinen auch Diene auf, insbesondere 1,3-Butadien, so kann in einem Vorschritt vor der eigentlichen Verfahrensstufe 1) der größte Teil des Butadiens entfernt werden. Die Diene können dabei z. B. durch Extraktion oder Extraktivdestillation vom Kohlenwasserstoffstrom I abgetrennt werden oder die im Kohlenwasserstoffstrom I enthaltenen Diene können z. B. bis zu einer Restkonzentration von ca. 2000 Massen-ppm selektiv zu linearen Butenen hydriert werden. Zurück bleibt in beiden Fällen ein Kohlenwasserstoffstrom I der kein bzw. maximal 2000 Massen-ppm an Dienen aufweist.

Als Verfahrensstufe 1) sind alle Verfahren geeignet, die die Aufkonzentrierung von Isobuten in Kohlenwasserstoffströmen ermöglichen. So kann die Verfahrensstufe 1) z. B. eine oder mehrere thermische Trennstufen aufweisen, bei denen andere Verbindungen vom Isobuten abgetrennt werden. Solche thermischen Trennstufen können z. B. Destillationen sein. So können aus einem Kohlenwasserstoffstrom I, der z. B. neben Isobuten, auch 1-Buten, 2-Buten, Butan und Isobutan aufweist, bis auf das 1-Buten alle anderen der genannten Begleitstoffe durch Destillation vom Isobuten abgetrennt werden. Beträgt das Massen-Verhältnis von 1-Buten zu Isobuten in einem solchen Kohlenwasserstoffstrom I beispielsweise 1 zu 3, genügt allein die Abtrennung aller weiteren Bestandteile aus dem Kohlenwasserstoffstrom I um einen Kohlenwasserstoffstrom II zu erhalten, der einen Anteil an Isobuten von zumindest 75 Massen-% in Bezug auf die enthaltenen Olefine aufweist.

In einer bevorzugten Ausführungsform des erfindungsgemäßen Verfahrens wird in der Verfahrensstufe 1) das im Kohlenwasserstoffstrom I enthaltenen Isobuten dadurch angereichert, dass das im Kohlenwasserstoffstrom I enthaltenen 1-Buten durch Isomerisierung, insbesondere durch Hydroisomerisierung zu cis- bzw. trans-2-Buten umgewandelt wird. Eine solche Hydroisomerisierung kann z. B. in einer Reaktivdestillationskolonne durchgeführt werden. Das bei der Isomerisierung erhaltene Reaktionsgemisch kann dann einfach thermisch, vorzugsweise durch Destillation, in eine Isobuten aufweisende Fraktion, die bei der Destillation als Kopfprodukt erhalten wird und eine 2-Butene aufweisende Fraktion, die bei der Destillation als Sumpffraktion erhalten wird, aufgetrennt werden. Ein als Verfahrensstufe 1) geeignetes Verfahren zur Hydroisomerisierung wird beispielsweise von der OXENO Olefinchemie GmbH in WO 03/035587 beschrieben.

Die so erhaltene Isobuten aufweisende Fraktion kann als Kohlenwasserstoffstrom II dem Verfahrenschritt a) des erfindungsgemäßen Verfahrens zugeführt werden. Der bei dieser Ausführungsform des erfindungsgemäßen Verfahrens erhaltene Kohlenwasserstoffstrom II weist vorzugsweise einen Anteil an Isobuten bezogen auf die Masse des Kohlenwasserstoffstroms II von zumindest 75 Massen-%, vorzugsweise von 75 bis 98 Massen-% und besonders bevorzugt von 80 bis 95 Massen-% auf.

Eine weitere Möglichkeit die Verfahrensstufe 1) auszuführen kann darin bestehen, Isobuten oder eine oder mehrere der im Kohlenwasserstoffstrom I enthaltenen Verbindungen chemisch zu Verbindungen umzusetzen, die sich leicht von anderen Bestandteilen im Kohlenwasserstoffstrom I abtrennen lassen. Das Abtrennen kann wiederum thermisch erfolgen oder z. B. mechanisch, wie z. B. durch Filtration, falls bei der Umsetzung ein Feststoff erhalten wird. Wird bei der Umsetzung das Isobuten zu einer Verbindung umgesetzt, so wird vorzugsweise eine solche Verbindung hergestellt, aus der Isobuten auf einfache Weise wieder erhalten werden kann.

Vorzugsweise wird in dem erfindungsgemäßen Verfahren eine Verfahrensstufe 1) durchgeführt, die die Verfahrensschritte
1a) Umsetzen von im Isobuten aufweisenden Kohlenwasserstoffstrom I enthaltenem Isobuten mit einer Verbindung V um ein Produkt P zu erhalten,
1b) Abtrennen des Produktes P vom Kohlenwasserstoffstrom I,
1c) Spaltung des Produktes P in Isobuten und Verbindung V und
1d) Abtrennen des Isobutens von der Verbindung V unter Erhalt eines Kohlenwasserstoffstroms II,
   aufweist.

Bei der Durchführung der Verfahrensschritte 1a) und/oder 1c) kann es vorteilhaft sein, wenn zumindest eine Reaktivdestillationskolonne eingesetzt wird. Durch den Einsatz einer Reaktivdestillationskolonne kann der Umsatz erhöht werden, indem während der Reaktion laufend Produkt aus dem Reaktionsgemisch entfernt wird und so das Gleichgewicht verschoben wird.

Vorzugsweise wird in dem Verfahrensschritt 1a) eine Verbindung V eingesetzt, die ausgewählt ist aus Wasser und Alkohol, insbesondere Methanol oder Ethanol. Bevorzugt wird durch die Umsetzung des im Kohlenwasserstoffstrom 1 enthaltenen Isobutens mit Wasser tert.-Butanol (TBA) hergestellt, welches auf einfache Weise z. B. thermisch, vorzugsweise durch Destillation, von dem Kohlenwasserstoffstrom I abgetrennt werden kann. Durch eine Spaltung von TBA kann daraus wieder Isobuten und Wasser gewonnen werden. Durch z. B. thermisches Abtrennen des Wassers vom Isobuten wird ein Kohlenwasserstoffstrom II erhalten, der fast ausschließlich Isobuten enthält. Die Herstellung von TBA aus Isobuten aufweisenden Kohlenwasserstoffströmen ist lange bekannt. Auch die Spaltung von TBA zu Isobuten und Wasser ist lange bekannt.

Die Umsetzung des Isobuten aufweisenden Kohlenwasserstoffstroms 1 mit Wasser kann z. B. wie in WO 99/33775, DE 30 25 262, DE 103 30 710 oder EP 1 616 848 und den dort zitierten Schriften erfolgen. Auf die vorgenannten Schutzrechte wird ausdrücklich verwiesen und deren Offenbarung soll vom Offenbarungsgehalt der vorliegenden Erfindung umfasst sein.

Die Umsetzung von Isobuten mit Wasser erfolgt bevorzugt bei einer Temperatur von 30 bis 120 °C, vorzugsweise bei einer Temperatur von 35 bis 70 °C. Erfolgt die Umsetzung in mehreren hintereinandergeschalteten Reaktoren, so kann es vorteilhaft sein, wenn diese mit unterschiedlichen Temperaturen betrieben werden. Beispielsweise kann bei vier hintereinander geschalteten Reaktoren der erste bei einer mittleren Temperatur von 67 bis 70 °C, der zweite bei einer von 53 bis 56 °C, der dritte bei einer von 42 bis 46 °C und der vierte Reaktor bei 42 bis 38 °C betrieben werden.

Die erfindungsgemäße Umsetzung von Isobuten mit Wasser kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 4 MPa. Um in den Reaktoren Verdampfungsprobleme zu vermeiden, sollte der Druck 0,2 bis 0,4 MPa höher als der Dampfdruck des Reaktionsgemisches sein.

Als Katalysatoren werden bei der TBA-Synthese bevorzugt saure Ionenaustauscherharze, besonders bevorzugt feste Ionenaustauscherharze mit Sulfonsäuregruppen eingesetzt. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, Lewatit K2611, Lewatit K2631, OC 1501, Lewatit K2671, Lewatit K2629 oder Lewatit K2431. Die Handelsnamen oder Teile davon können eingetragene Marken der Hersteller sein.

Im erfindungsgemäßen Verfahren werden die Ionenaustauscherharze bei der TBA-Synthese bevorzugt in ihrer H-Form verwendet. Die Ionenaustauscherkapazität beträgt dabei vorzugsweise von 2 bis 7, insbesondere 3 bis 6 eq/kg (bezogen auf feuchtes handelsübliches Harz).

Ganz besonderes bevorzugt werden makroporöse Harze, wie beispielsweise Lewatit SCP 118, Lewatit SCP 108, Lewatit K2631, Amberlyst 15 oder Amberlyst 35, eingesetzt. Optional können die Ionenaustauscherharze als Formkörper, wie beispielsweise Zylinder, Ringe oder Kugeln, eingesetzt werden. Die Korngröße der technischen Harze liegt im Allgemeinen zwischen 0,5 und 2 mm. Die Korngrößenverteilung kann aber auch enger oder weiter gewählt werden. So können beispielsweise auch Ionenaustauscherharze mit sehr einheitlicher Korngröße (monodisperse Harze) eingesetzt werden.

Die Abtrennung des TBA gemäß Schritt b), welches in Verfahrensschritt 1a) als Produkt P erhalten wurde, kann z. B. thermisch erfolgen. Vorzugsweise wird das aus dem Verfahrensschritt 1a) erhaltene Reaktionsgemisch einer Destillationskolonne zugeführt, die vorzugsweise bei oder unter dem Druck der TBA-Synthese, jedoch mindestens bei einem Druck von über 0,1 MPa arbeitet. Bei der Destillation fällt als Kopfprodukt ein Kohlenwasserstoffgemisch an, das aus nicht umgesetztem Isobuten und aus den mit dem Edukt eingebrachten "inerten" Kohlenwasserstoffen besteht. Als Sumpfprodukt wird eine wässrige tert.-Butanol-Lösung gewonnen. Die Ausgestaltung und der Betrieb solcher Destillationen gehört zum Können des Durchschnittsfachmanns und wird hier nicht näher erläutert.

Wird der Verfahrensschritt 1a) unter Verwendung einer Reaktivdestillationskolonne durchgeführt, kann der Verfahrensschritt 1b) in dem Verfahrensschritt 1a) integriert sein bzw. zeitgleich mit Verfahrensschritt 1a) in der Reaktivdestillationskolonne durchgeführt werden.

Das abgetrennte Kohlenwasserstoffgemisch kann zu weiteren Wertprodukten aufgearbeitet werden. Wurde beispielsweise Raffinat I oder selektivhydrierter C₄-Schnitt als Edukt eingesetzt, so enthält das Kopfprodukt neben nicht umgesetztem Isobuten, lineare Butene sowie Isobutan und n-Butan. Aus diesem Gemisch kann das restliche Isobuten z. B. durch Umsetzung mit Alkohol zu Alkyl-tert.-butylether abgetrennt werden. Im verbleibenden Raffinat können die linearen Butene - optional nach Abtrennung von 1-Buten - zu Di-n-buten und ihren höheren Oligomeren umgesetzt werden. Eine andere Verwendung des isobutenfreien Gemisches ist die Aufarbeitung zu reinem 1-Buten.

Ein Teil der gewonnenen wässrigen tert.-Butanol-Lösung kann in den Prozess zurückgefahren (rezykliert) werden. Der andere Teil kann als solcher in Verfahrensschritt 1c) verwendet werden oder auf reines tert.-Butanol und einem Azeotrop aus Wasser und tert.-Butanol z. B. thermisch aufgearbeitet werden.

Die Spaltung des TBA gemäß Verfahrensschritt 1c) kann auf an und für sich bekannte Weise erfolgen. Die Spaltung kann in der Flüssigphase an sauren Katalysatoren, insbesondere an sauren Ionenaustauscherharzen, durchgeführt werden. Ein solches Verfahren beschreibt US 4,423,271. Die Spaltung kann aber auch in der Gasphase durchgeführt werden. Ein solches Verfahren, bei dem die Spaltung an sauren Aluminiumoxiden erfolge, wird z. B. in US 3,665,048 beschrieben. Bevorzugt erfolgt die TBA-Spaltung in zumindest einer Reaktivdestillationskolonne. Ein solches Verfahren wird in EP 0 726 241 beschrieben. Besonders bevorzugt erfolgt die TBA-Spaltung in zumindest einem Festbettreaktor, der quasi isotherm betrieben wird. Ein solches Verfahren wird in EP 1 489 062 beschrieben. Auf die vorgenannten Schutzrechte wird ausdrücklich verwiesen und deren Offenbarung soll zum Offenbarungsgchalt der vorliegenden Erfindung gehören.

Der Verfahrensschritt 1c) des erfindungsgemäßen Verfahren wird bei der Spaltung von TBA vorzugsweise bei einer Temperatur von 80 bis 150 °C, bevorzugt 100 bis 120°C durchgeführt. Der Druck beträgt bei der TBA-Spaltung vorzugsweise von 0,5 bis 2,5 MPa, wobei der Druck bevorzugt so hoch gewählt wird, dass das in den Reaktoren, bzw. in den Reaktionszonen der Reaktoren entstehende Isobuten ohne Ausbildung einer Gasphase nahezu vollständig und im

Wesentlichen homogen im Reaktionsgemisch gelöst bleibt.

Als Katalysatoren werden bei der TBA-Spaltung bevorzugt saure Ionenaustauscherharze, wie sie oben für die TBA-Herstellung beschrieben wurden, eingesetzt.

Die Abtrennung des Isobutens gemäß Verfahrensschritt 1d) kann wie in US 4,423,271, US 3,665,048, EP 0 726 241 oder EP 1 489 062 beschrieben erfolgen. Bevorzugt erfolgt die Wasserabtrennung wie in EP 1 489 062 beschrieben in zumindest zwei Kolonnen. Die Aufarbeitung des aus der TBA-Spaltung erhaltenen Reaktionsgemisches erfolgt vorzugsweise dadurch, dass aus dem Reaktoraustrag in einer ersten Kolonne über Kopf der überwiegende Teil des Isobutens als Isobuten/Wasser-Azeotrop abgetrennt wird. Aus dem Kopfprodukt der ersten Kolonne kann nach einer Abtrennung eines Teils des Wassers, z. B. durch Phasentrennung, durch azeotrope Destillation in einer zweiten Kolonne ein nahezu wasserfreies Isobuten gewonnen werden. Die Ausgestaltung und der Betrieb solcher Destillationen gehört zum Können des Durchschnittsfachmanns und wird hier nicht näher erläutert.

Das so erhaltene Isobuten kann als Kohlenwasserstoffstrom II dem Verfahrenschritt a) des erfindungsgemäßen Verfahrens zugeführt werden. Der bei dieser Ausführungsart des erfindungsgemäßen Verfahrens erhaltene Kohlenwasserstoffstrom II weist vorzugsweise einen Anteil an Isobuten bezogen auf die Masse des Kohlenwasserstoffstroms II von zumindest 85 Massen-%, vorzugsweise von 90 bis 99,99 Massen-% und besonders bevorzugt von 95 bis 99,8 Massen-% auf.

In einer besonders bevorzugten Ausführungsform des erfindungsgemäßen Verfahrens wird in der Verfahrensstufe 1) das im Kohlenwasserstoffstrom I enthaltenen Isobuten durch die Umsetzung des im Kohlenwasserstoffstrom I enthaltenen Isobutens mit Alkohol zu Alkyl-tert.-butylether (ATBE), welcher auf einfache Weise z. B. thermisch, vorzugsweise durch Destillation, vom Kohlenwasserstoffstrom I abgetrennt werden kann. Durch eine Spaltung des ATBE kann daraus wieder Isobuten und Alkohol gewonnen werden. Durch z. B. thermisches Abtrennen des Alkohols vom Isobuten wird dann ein Kohlenwasserstoffstrom II erhalten, der fast ausschließlich Isobuten enthält. Die Herstellung von ATBE, insbesondere von Methyl-tert.-butylether (MTBE) und Ethyl-tert.-butylether (ETBE) aus Isobuten aufweisenden Kohlenwasserstoffströmen durch Umsetzung dieser mit Methanol bzw. Ethanol ist lange bekannt. Auch die Spaltung von ATBE, insbesondere von MTBE oder ETBE zu Isobuten und den entsprechenden Alkoholen ist lange bekannt.

Für die Umsetzung von Isobuten mit Alkoholen, insbesondere mit Methanol zu Methyl-tert.-butylether wurden diverse Verfahrensvarianten entwickelt (vergleiche: Ullmann's Encyclopedia of Industrial Chemistry, Online Version, 2004, Wiley & Sons, Stichwort Methyl-tert.-butylether, und dort zitierte Literatur; Obenaus, Fritz; Droste, Wilhelm, Erdoel & Kohle, Erdgas, Petrochemie (1980), 33(6), 271-275; DE 26 29 769; DE 28 53 769). Die Dokumente EP 0 048 893 und DE 25 21 964 beschreiben Verfahren zur Herstellung von ATBE. In den Dokumenten US 3,726,942, US 3,846,088, US 4,334,964, US 4,544,776, DE 30 15 882 und WO 2004/007412 werden Verfahren zur Herstellung von MTBE beschrieben und die Dokumente EP 0 071 032, DE 10 2005 062699 und DE 10 2005 062722 beschreiben Verfahren zur Herstellung von ETBE. Alle die genannten Verfahren können als Verfahrensschritt 1a) in dem erfindungsgemäßen Verfahren eingesetzt werden. Auf die vorgenannten Schutzrechte wird ausdrücklich verwiesen und deren Offenbarung soll zum Offenbarungsgehalt der vorliegenden Erfindung gehören.

Die Umsetzung des Isobutens im Kohlenwasserstoffstrom I in Verfahrensschritt 1 a) kann in einem oder mehreren Reaktor(en) durchgeführt werden. Vorzugsweise werden zumindest zwei Festbettreaktoren eingesetzt. Als Reaktoren können herkömmliche Festbettreaktoren, wie z. B. Rohrbündelreaktoren, adiabatische Festbettreaktoren oder Kreislaufreaktoren, eingesetzt werden. Sie können mit oder ohne partielle Rückführung, wobei gegebenenfalls der Rückführungsstrom gekühlt werden kann, betrieben werden.

Die Reaktoren werden vorzugsweise bei Temperaturen von 10 bis 160 °C, bevorzugt von 30 bis 110 °C und besonders bevorzugt von 60 bis 80 °C betrieben. Der Druck in den Reaktoren, insbesondere in den Festbettreaktoren beträgt vorzugsweise von 0,5 bis 5 MPa, bevorzugt von 1 bis 2 MPa.

Das molare Verhältnis von Alkohol zu Isobuten beträgt vorzugsweise von 10 zu 1 bis 0,9 zu 1, bevorzugt von 5 zu 1 bis 1 zu 1 und besonders bevorzugt von 2 zu 1 bis 1 zu 1.

Als Katalysator wird bei der Herstellung von ATBE in dem Verfahrensschritt 1a) vorzugsweise ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen keine sauren Stoffe an das Produktgemisch abgeben, weil dies zu Ausbeuteverluste führen kann. Die Aktivität der Katalysatoren wird vorzugsweise so gewählt, dass sie unter Reaktionsbedingungen die Addition von Alkohol an Isobuten katalysieren, jedoch kaum die Addition an lineare Butene. Weiterhin sollten die Katalysatoren die Oligomerisierung von linearen Butenen und die Dialkyletherbildung aus zwei Molekülen eingesetzten Alkohols möglichst nicht oder nur wenig katalysieren.

Als feste Katalysatoren können beispielsweise Zeolithe, säureaktivierte Bentonite und/oder Tonerden, sulfonierte Zirkoniumoxide, Montmorillonite oder saure Ionenaustauscherharze verwendet werden. Eine im erfindungsgemäßen Verfahren bevorzugte Gruppe von sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere können die Cooligomeren, die durch Umsetzung von Styrol mit Divinylbenzol entstehen, als Vorstufe für die Herstellung von Ionenaustauscherharzen mit Sulfonsäuregruppen verwendet werden. Die Harze können gelförmig, makroporös oder schwammförmig hergestellt werden. Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können in Verfahrensschritt 1a) bei der Herstellung von ATBE die Ionenaustauscherharze in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, bevorzugt von 0,7 bis 2,0 eq/l, insbesondere von 1,1 bis 2,0 eq/l bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

In einer bevorzugten Ausführungsform des Verfahrensschrittes 1a) wird die Addition des Alkohols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Verfahrensschritt 1a) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus dem Kohlenwasserstoffstrom I und dem Alkohol ein Reaktionsgemisch hergestellt, das hinsichtlich seiner Isobuten-, Alkohol- und tert.-Butylether-Konzentration in der Nähe des thermodynamischen Gleichgewichts liegt. Der Umsatz des Isobutens beträgt dabei bevorzugt größer 90 %. Dieses Gemisch wird in der nächsten/letzten Reaktionsstufe in die Reaktivdestillationskolonne eingespeist, wo ein weiterer Teil des Isobutens zum Ether umgesetzt wird.

Die Reaktivdestillationskolonne kann vorzugsweise bei Drücken, gemessen am Kolonnenkopf, von 0,3 bis 2,5 MPa, bevorzugt 0,5 bis 1,5 MPa und besonders bevorzugt von 0,7 bis 1,0 MPa betrieben werden. Die Umsetzung des Isobutens mit Alkohol zum entsprechenden tertiären Butylether erfolgt in der Reaktivdestillation vorzugsweise bei einer Temperatur von 10 bis 140 °C, bevorzugt von 40 bis 90 °C und besonders bevorzugt von 60 bis 80 °C (Temperatur im Bereich der Kolonne, in der sich der Katalysator befindet. Die Sumpftemperatur der Kolonne kann deutlich höher liegen).

Das aus dem Verfahrensschritt 1a) erhaltene Reaktionsgemisch wird zur Abtrennung des ATBE als Produktes P vom restlichen Kohlenwasserstoffstrom I dem Verfahrensschritt 1b) zugeführt. Es kann vorteilhaft sein, das ATBE in Verfahrensschritt 1b) möglichst vollständig von Verunreinigungen, insbesondere von Olefinen oder Kohlenwasserstoffen zu befreien.

Der Verfahrensschritt 1b) kann als unabhängiger Verfahrensschritt ausgeführt sein. Vorzugsweise wird der Verfahrensschritt 1a) thermisch, insbesondere als ein- oder mehrstufige Destillation durchgeführt. Die Ausgestaltung und der Betrieb solcher Destillationen gehört zum Können des Durchschnittsfachmanns und wird hier nicht näher erläutert.

Der Verfahrensschritt 1b) kann aber auch bereits im Verfahrensschritt 1a) enthalten sein, wenn in dem Verfahrensschritt 1a) als letzte Reaktionsstufe eine Reaktivdestillation eingesetzt wird. In dem Fall wird als Sumpfprodukt der Destillationskolonne ATBE erhalten, welcher gegebenenfalls über eine oder mehrere weitere Destillationen aufgereinigt werden kann. In diesem Fall wird der Verfahrensschritt 1b) zeitgleich mit dem Verfahrensschritt 1a) in der Reaktivdestillationskolonne durchgeführt. Der ATBE wird als Sumpfprodukt der Reaktivdestillationskolonne erhalten und kann durch weitere Destillationsschritte weiter aufgearbeitet werden. Der restliche Kohlenwasserstoffstrom I ist bei der Reaktivdestillation im Wesentlichen im Kopfprodukt enthalten.

Eine vollständige Abtrennung des ATBE vom eingesetzten Alkohol ist in Verfahrensschritt 1b) nicht zwingend notwendig. Vielmehr ist es möglich, das nach der Abtrennung des restlichen Kohlenwasserstoffstroms I erhaltene ATBE, also insbesondere ETBE oder MTBE, welches noch Alkohol enthält, direkt in Schritt 1c) einzusetzen. Auf diese Weise kann auf eine aufwändige Reinigungsstufe verzichtet werden.

Die Spaltung von ATBE wie z. B. MTBE oder ETBE gemäß Verfahrensschritt 1c) zur Gewinnung von Isobuten kann generell in zwei unterschiedlichen Varianten erfolgen. Zum einen kann die Spaltung in der Flüssigphase an sauren Ionentauscherharzen wie beispielsweise in DE 35 09 292 A1 bzw. DE 36 10 704 A1 beschrieben oder an sauren Aluminiumoxiden wie beispielsweise in DD 240 739 A1 offenbart, ausgeführt werden. Zum anderen kann die Spaltungsreaktion in der Gas-/Flüssigphase in einer Art kombinierter Reaktionsdestillationskolonne an sauren Katalysatoren durchgeführt werden, so offenbart in EP 0 302 336 A1 oder DE 4 322 712. In EP 0 302 336 A1 wird die Abspaltung von Methanol aus MTBE an einem sauren Ionentauscherharz, das im Kolonnensumpf positioniert ist, beschrieben. In DE 4 322 712 wird der tertiäre Ether oberhalb der Reaktionszone einer Reaktionsdestillationskolonne zugeführt, wobei der Verstärkerteil der Kolonne zur Isobutenreinigung dient, während im Abtriebsteil der Kolonne Methanol vom MTBE-Methanol-Azeotrop abgetrennt wird. Bevorzugt erfolgt die Spaltung des ATBE wie in EP 1 149 814 und die Spaltung von MTBE wie in WO 04/018393 und den dort zitierten Schriften beschrieben. Eine weitere, wenn auch weniger bevorzugte Möglichkeit, ist die Durchführung der ATBE-Spaltung in der Gasphase. Alle die genannten Verfahren können als Verfahrensschritt 1c) in dem erfindungsgemäßen Verfahren eingesetzt werden. Auf die vorgenannten Schutzrechte wird ausdrücklich verwiesen und deren Offenbarung soll zum Offenbarungsgehalt der vorliegenden Erfindung gehören.

Die ATBE-Spaltung kann in einem oder mehreren Reaktoren durchgeführt werden. Bei Verwendung von mehreren Reaktoren können diese in Reihe oder parallel oder sowohl in Reihe als auch parallel miteinander verschaltet sein. Es können verschiedene Reaktortypen eingesetzt werden, wie beispielsweise Festbettreaktoren oder Rohrbündelreaktoren oder Kettlereaktoren. Der/die Reaktor(en) kann/können isotherm, polytrop oder adiabatisch, im geraden Durchgang oder mit externem Recycle betrieben werden.

Der Verfahrensschritt 1c) wird bei der Spaltung von ATBE, insbesondere von MTBE oder ETBE vorzugsweise bei einer Temperatur von 60 bis 200 °C, bevorzugt 80 bis 125 °C und besonders bevorzugt bei einer Temperatur von 105 bis 115 °C durchgeführt. Bei Verwendung mehrerer Reaktoren können die Temperaturen unabhängig voneinander gleich oder verschieden sein. Der Druck, bei der der Verfahrensschritt 1c) durchgeführt wird, beträgt vorzugsweise von 0,1 bis 1,0 MPa, bevorzugt bei 0,3 bis 0,7 MPa. Als Katalysator wird vorzugsweise ein Kationenaustauscherharz, insbesondere ein Kateionenaustauscherharz mit sulfonsauren Gruppen eingesetzt.

In einer bevorzugten Ausführungsform des Verfahrensschrittes 1c) wird die Spaltung des ATBE in Gegenwart eines sauren Katalysators so durchgeführt, dass zumindest eine Reaktionsstufe als Reaktivdestillation durchgeführt wird. Besonders bevorzugt wird die Spaltung in Verfahrensschritt 1c) in mindestens zwei Reaktionsstufen durchgeführt, wobei vorzugsweise zumindest eine, besonders bevorzugt die letzte Reaktionsstufe als Reaktivdestillation durchgeführt wird.

Der bevorzugte Arbeitsbereich der Reaktivdestillation liegt bei einem Druck zwischen 0,1 und maximal 1 MPa, vorzugsweise bei einem Druck von 0,3 bis 0,7 MPa. Wird als Katalysator ein z. B. Kationenaustauscherharz verwendet, ist bei über 125 °C mit einer erheblichen Abspaltung sulfonsaurer Gruppen von der Harzoberfläche zu rechnen, sodass allmählich eine Desaktivierung des Katalysators eintritt. Hier empfiehlt es sich eine Reaktionstemperatur von 105 bis 115 °C nicht zu überschreiten.

Als Katalysator können bei der ATBE-Spaltung im erfindungsgemäßen Verfahrensschritt 1c) insbesondere solche Ionenaustauscherharze, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinylbenzol-Typs werden u. a. unter folgenden Handelsnamen verkauft: Duolite C20, Duolite C26, Amberlyst A15, Amberlyst A35, Amberlyst 36, Amberlite IR-120, Amberlite 200, Dowex 50, Lewatit K2431, Lewatit K2441, Lewatit K2621, Lewatit K2629, Lewatit K2641.

Die Eigenschaften dieser Harze, insbesondere spezifische Oberfläche, Porosität, Stabilität, Quellung bzw. Schrumpfung und Austauschkapazität, können durch den Herstellprozess variiert werden. Optional können auch kommerzielle, makroporöse Kationenaustauscherharze, die durch partiellen Ionenaustausch oder durch thermische Desulfonierung modifiziert sind, eingesetzt werden.

Wird im erfindungsgemäßen Verfahrensschritt 1c) eine Reaktivdestillation durchgeführt, kommen bevorzugt strukturierte katalytische Mehrzweckpackungen zum Einsatz, wie sie beispielsweise in US 5 348 710, EP 0 950 433, EP 0428 265, EP 433 222 beschrieben sind. Derartige strukturierte Packungen im Sinne des erfindungsgemäßen Verfahrens sind beispielsweise im Handel erhältlich als Katapak^{®} der Sulzer AG, Katamax^{®} der Firma Koch-Glitsch oder Multipak^{®} der Montz GmbH. Diese werden z. B. aus Blechen, bevorzugt aus Schwarzstahl, Edelstahl, Hastelloy, Kupfer oder Aluminium oder strukturierten Gewebebahnen hergestellt.

Das aus dem Verfahrensschritt 1c) erhaltene Reaktionsgemisch wird zur Abtrennung des Isobutens von der Verbindung V dem Verfahrensschritt 1d) zugeführt.

Der Verfahrensschritt 1d) kann als unabhängiger Verfahrensschritt ausgeführt sein. In dem Fall wird er bevorzugt thermisch, insbesondere als ein- oder mehrstufige Destillation durchgeführt. Die Ausgestaltung und der Betrieb solcher Destillationen gehört zum Können des Durchschnittsfachmanns und wird hier nicht näher erläutert.

Der Verfahrensschritt 1d) kann aber auch bereits im Verfahrensschritt 1c) enthalten sein, wenn in dem Verfahrensschritt 1c) als letzte Reaktionsstufe eine Reaktivdestillation eingesetzt wird. In dem Fall wird als Kopfprodukt der Destillationskolonne Isobuten erhalten, welcher gegebenenfalls über eine oder mehrere weitere Destillationen aufgereinigt werden kann.

Der aus dem Verfahrensschritt 1d) erhaltene Kohlenwasserstoffstrom II kann als Ausgangsprodukt in dem Verfahrensschritt a) des erfindungsgemäßen Verfahrens eingesetzt werden. Der bei dieser Ausführungsart des erfindungsgemäßen Verfahrens erhaltene Kohlenwasserstoffstrom II weist vorzugsweise einen Anteil an Isobuten bezogen auf die Masse des Kohlenwasserstoffstroms II von zumindest 85 Massen-%, vorzugsweise von 90 bis 99,99 Massen-% und besonders bevorzugt von 95 bis 99,8 Massen-% auf.

### Verfahrensstufe a) (Hydroformylierung)

Im Verfahrenschritt a) wird ein Kohlenwasserstoffstrom II, der zumindest 70 Massen-% Isobuten in Bezug auf die im Kohlenwasserstoffstrom enthaltenen Olefine aufweist und der aus dem Kohlenwasserstoffstrom I erhalten wurde oder mit diesem identisch ist, einer Hydroformylierung zugeführt, in der Isobuten in Gegenwart eines Rhodium-Katalysators hydroformyliert wird.

Die Hydroformylierung des Isobutens im Kohlenwasserstoffstrom II kann in einer Stufe einer oder mehreren Stufen erfolgen. Sind im Kohlenwasserstoffstrom neben Isobuten weitere Olefine vorhanden, so wird für die Hydroformylierung ein Katalysator benötigt, der geeignet ist, die Doppelbindung von Isobuten zu hydroformylieren.

Die im Kohlenwasserstoffstrom II möglicherweise vorliegenden Olefine unterscheiden sich in ihrer Reaktivität bei der Hydroformylierung beträchtlich. Im Allgemeinen sind Olefine mit endständigen Doppelbindungen reaktiver als Olefine mit mittelständigen Doppelbindungen und lineare Olefine reaktiver als verzweigte. 1-Buten ist somit reaktiver als Isobuten und Isobuten ist reaktiver als die beiden 2-Butene. Diese unterschiedliche Reaktivität kann genutzt werden, um einen hohen Anteil an Produkten, die durch endständige Hydroformylierung entstanden sind, zu gewinnen, d. h., aus Isobuten wird im Wesentlichen 3-Methylbutanal und nicht 2,2-Dimethylpropanal gewonnen. Dieser Zusammenhang kann im vorliegenden Verfahren dahingehend genutzt werden, dass bei der Hydroformylierung nur die α-Olefine (1-Buten, Isobuten), insbesondere das Isobuten jedoch nicht die 2-Butene zu den entsprechenden Aldehyden umgesetzt werden. Gegebenenfalls nicht umgesetzte 2-Butene können aus dem aus Verfahrensschritt a) erhaltenen Reaktionsgemisch abgetrennt und einer weiteren Verwendung zugeführt werden.

Die Hydroformylierung gemäß Verfahrensschritt a) wird vorzugsweise unter solchen Bedingungen durchgeführt, dass das Isobuten möglichst selektiv zu 3-Methylbutanal umgesetzt wird. Als Katalysator wird in Verfahrensschritt a) deshalb vorzugsweise ein Rhodium-Komplexkatalysator eingesetzt, der Phosphor-organische Liganden aufweist.

Als Katalysatoren können beispielsweise Verbindungen eingesetzt werden, die Rhodium und triwertige organische Phosphorverbindungen, insbesondere Phosphine oder Phosphite als Liganden aufweisen. Die Umsetzung kann bei Verwendung von Phosphinen als Katalysatorkomponente in homogener Phase (analog dem UCC-Verfahren EP 0 562 451) oder in heterogener Phase (analog dem Rhone-Poulenc-Ruhrchentie-Verfahren DE 026 27 354, EP 0 562 451) durchgeführt werden. Wegen der einfacheren Umsetzung wird der Verfahrensschritt a) bevorzugt gemäß dem ersten Verfahren durchgeführt.

Die Reaktionstemperaturen bei der Hydroformylierung betragen bevorzugt von 70 bis 150 °C, vorzugsweise von 100 bis 130 °C. Die Verfahrensdrücke betragen vorzugsweise von 2 bis 20 MPa, bevorzugt von 3 bis 6 MPa. Als Hydroformylierungsagens wird vorzugsweise ein Gemisch aus Kohlenmonoxid und Wasserstoff im molaren Verhältnis von Kohlenmonoxid zu Wasserstoff von 1 zu 10 bis 10 zu 1, bevorzugt im molaren Verhältnis von 1,1 zu 1 bis 1 zu 1,1 und besonders bevorzugt 1 zu 1 eingesetzt. Die Rhodiumkonzentration im Hydroformylierungsgemisch beträgt vorzugsweise von 5 bis 500 Massen-ppm, vorzugsweise 10 bis 200 Massen-ppm. Je Mol Rhodium werden vorzugsweise von 1 bis 50 Mol, bevorzugt 5 bis 30 Mol phosphor-organischer Ligand eingesetzt. Die Umsetzung kann kontinuierlich oder diskontinuierlich durchgeführt werden. Bevorzugt ist aber eine kontinuierliche Arbeitsweise.

Geeignete Katalysatoren sind beispielsweise auch Rhodium-Komplexkatalysatoren, die ein- oder mehrzähnige Phosphitliganden enthalten. Als Liganden bzw. Komplexkatalysatoren können insbesondere solche eingesetzt werden, wie sie in EP 0 155 508 (UCC), EP 0 213 639 (UCC), EP 0 214 622 (UCC), EP 0 471 071 (BASF) EP 1 099 677 (OXENO), EP 1 099 678 (OXENO) oder EP 1 201 675 (OXENO) beschrieben worden sind. Besonders gut geeignete Rhodium-Komplexkatalysatoren mit einzähnigen Phosphitliganden sind beispielsweise Triarylphosphite, deren Arylgruppen sowohl in ortho-Stellung zum Phosphit-Sauerstoff eine sperrige Gruppe aufweisen als auch in m- oder p-Stellung substituiert sind, wie z. B. Tris(2,4-di-tert.-butyl-phenyl)phosphit. Ein solches Tris(2,4-di-tert.-butyl-phenyl)phosphit ist beispielsweise unter dem Handelsnamen ALKANOX 240 von der Great Lakes Chemical Corporation zu beziehen. Die Hydroformylierung von Isobuten unter Verwendung eines Katalysatorsystems, das aus Rhodium und einem Bisphosphit besteht, wird beispielsweise in den Patentschriften US 4,668,651, US 4,769,498 und WO 85/03702 beschrieben. Auf alle genannten Schriften wird ausdrücklich verwiesen und deren Offenbarungsgehalt soll Gegenstand der vorliegenden Beschreibung sein. Besonders bevorzugt wird die Hydrofomrylierung in Gegenwart eines einzähnigen Phosphit-Liganden, insbesondere in Gegenwart von Tris(2,4-di-tert.-butylphenyl)phosphit als Ligand durchgeführt.

Optional kann die Hydroformylierung der 1-Olefine im Mehrphasensystem, wobei Edukt, Produkt und Synthesegas in einer kontinuierlichen Katalysatorphase dispergiert sind, unter hohen Leerrohrgeschwindigkeiten durchgeführt werden. Solche Verfahren werden beispielsweise in DE 199 25 384 A1 und DE 199 57528 A1 beschrieben, auf welche hier ausdrücklich verwiesen wird.

Die Hydroformylierung der α-Olefine kann einstufig oder zweistufig durchgeführt werden. Die zweistufige Durchführung der Hydroformylierung ist insbesondere dann vorteilhaft, wenn der Kohlenwasserstoffstrom II mehr als 5 Massen-%, vorzugsweise mehr als 10 Massen-% und insbesondere zwischen 10 und 25 Massen-% 1-Buten bezogen auf die im Kohlenwasserstoffstrom II enthaltenen Olefine aufweist. Bei der zweistufigen Hydroformylierung wird im ersten Reaktor vorwiegend 1-Buten und im zweiten Reaktor im Wesentlichen Isobuten umgesetzt. In beiden Reaktoren können die gleichen Katalysatoren oder unterschiedliche eingesetzt werden. Bei Verwendung gleicher Katalysatoren ist eine gemeinsame Katalysatoraufarbeitung möglich.

Nach der gerade beschriebenen Hydroformylierung von Isobuten und gegebenenfalls von 1-Buten im Verfahrensschritt a) verbleiben im Kohlenwasserstoffstrom II gegebenenfalls vorhandene 2-Butene oder gesättigte Kohlenwasserstoffe und gegebenenfalls nicht umgesetztes Isobuten und/oder 1-Buten. Dieses Gemisch kann von den erhaltenen Aldehyden abgetrennt und einer weiteren Verwendung, z. B. ebenfalls einer Hydroformylierung zugeführt werden.

Optional kann dieses Gemisch, das als Olefine hauptsächlich 2-Butene enthält, zu überwiegend C₈-Olefinen, z. B. unter Verwendung von Nickel-haltigen Festbettkatalysatoren, oligomerisiert werden.

Aus den in Verfahrensschritt a) erhaltenen Hydroformylierungsgemischen kann nach bekannten Verfahren der Katalysator abgetrennt werden. Beispielsweise kann bei Verfahren, bei denen der Rhodium-Katalysator homogen im Reaktionsgemisch vorliegt, der Katalysator thermisch, z. B. destillativ abgetrennt werden. Bei der Umsetzung in heterogener Phase (zwei flüssige Phasen) kann die Abtrennung des Katalysators z. B. durch Phasentrennung erfolgen (Ed. B. Comils, W. A. Herrmann, Applied Homogeneous Catalysis with Organic Compounds, Vol. 1, S. 80, VCH-Verlag, 1996).

Die Hydroformylierungsgemische können vorzugsweise nach einer Entkatalysierung entweder direkt in Verfahrensstufe b) eingesetzt werden oder aber destillativ oder mit anderen Trennmethoden in zwei oder mehrere Fraktionen aufgetrennt werden. Insbesondere kann es vorteilhaft sein, das Hydroformylierungsgemisch so aufzuarbeiten, dass eine oder mehrere im Wesentlichen Aldehyde aufweisende Fraktionen erhalten werden.

### Verfahrensschritt b) (Hydrierung)

Die Hydroformylierungsgemische, die vorzugsweise entkatalysiert sind, oder die daraus durch ein Trennverfahren wie z. B. Destillation abgetrennten Aldehyde oder Aldehyd aufweisenden Fraktionen werden im Verfahrensschritt b) des erfindungsgemäßen Verfahrens hydriert. Durch die Hydrierung entstehen aus den Aldehyden die entsprechenden gesättigten Alkohole, insbesondere entsteht aus dem in Verfahrensschritt a) aus Isobuten erhaltenen 3-Methylbutanal der Alkohol 3-Methylbutanol.

Zur Hydrierung können im Verfahrensschritt b) als Katalysator z. B. Nickel-, Kupfer-, Kupfer/Nickel-, Kupfer/Chrom-, Kupfer/Chrom/Nickel-, Zink/Chrom-, Nickel/Molybdän-Katalysatoren eingesetzt werden. Die Katalysatoren können trägerfrei sein, oder die hydrieraktiven Stoffe bzw. ihre Vorläufer können auf Trägern, wie beispielsweise Siliziumdioxid oder Aluminiumoxid, aufgebracht sein. Bevorzugte Katalysatoren, an denen die Hydroformylierungsgemische hydriert werden, weisen jeweils 0,3 bis 15 Massen-% Kupfer und Nickel sowie als Aktivatoren 0,05 bis 3,5 Massen-% Chrom und vorteilhaft 0,01 bis 1,6 Massen-%, vorzugsweise 0,02 bis 1,2 Massen-% einer Alkalikomponente auf einem Trägermaterial, vorzugsweise Aluminiumoxid und Siliziumdioxid auf. Geeignete Hydrierkatalysatoren werden beispielsweise in EP 0 326 674 beschrieben. Die Mengenangaben beziehen sich auf den noch nicht reduzierten Katalysator. Die Alkalikomponente ist optional. Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudaten oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird vorzugsweise bei einem Gesamtdruck von 0,5 bis 50 MPa, bevorzugt von 1,5 bis 10 MPa durchgeführt. Eine Hydrierung in der Gasphase kann auch bei niedrigeren Drücken durchgeführt werden, wobei dann entsprechend große Gasvolumina vorliegen. Werden mehrere Hydrierungsreaktoren eingesetzt, können die Gesamtdrücke in den einzelnen Reaktoren innerhalb der genannten Druckgrenzen gleich oder verschieden sein. Die Reaktionstemperaturen können bei der Hydrierung in Verfahrensschritt b) in flüssiger oder gasförmiger Phase in der Regel von 120 bis 220 °C, insbesondere von 140 bis 180 °C betragen. Solche Hydrierungen sind beispielsweise in den Patentanmeldungen DE 198 42 369 und DE 198 42 370 beschrieben, auf welche hier ausdrücklich verwiesen wird.

Im erfindungsgemäßen Verfahren wird die Hydrierung bevorzugt in Gegenwart von Wasser durchgeführt. Das benötigte Wasser kann im Reaktorzulauf enthalten sein. Es ist jedoch auch möglich, Wasser an geeigneter Stelle in die Hydrierapparatur einzuspeisen. Bei Gasphasenhydrierung wird Wasser zweckmäßig in Form von Wasserdampf zugeführt. Ein bevorzugtes Hydrierverfahren ist die Flüssigphasenhydrierung unter Zusatz von Wasser, wie sie beispielsweise in DE 100 62 448 beschrieben ist. Besonders bevorzugt wird Hydrierung bei einem Wassergehalt von 0,05 bis 10 Massen-%, insbesondere 0,5 bis 5 Massen-%, ganz besonders 1 bis 2,5 Massen-% durchgeführt. Der Wassergehalt wird dabei im Hydrieraustrag bestimmt.

Die aus der Hydrierung erhaltenen Gemische können entweder direkt in Verfahrensstufe c) eingesetzt werden oder aber destillativ oder mit anderen Trennmethoden in zwei oder mehrere Fraktionen aufgetrennt werden. Insbesondere kann es vorteilhaft sein, das Hydrierungsgemisch so aufzuarbeiten, dass eine oder mehrere im Wesentlichen Alkohole mit der gleichen Anzahl an Kohlenstoffatomen aufweisende Fraktionen erhalten werden.

Wenn ausgehend von einem C₄-Kohlenwasserstoffschnitt ein Teil der darin enthaltenen linearen Olefine innenständig hydroformyliert wird, kann es zweckmäßig sein, das daraus durch Hydrierung entstandene 2-Methylbutanol ganz oder teilweise abzutrennen.

### Verfahrensschritt c) (Wasserabspaltung)

Aus dem nach der Hydrierung gemäß Verfahrensschritt b) erhaltenen Alkoholgemisch bzw. den crhaltenen Alkoholen oder Alkohol aufweisenden Fraktionen werden in dem Verfahrensschritt c) durch Wasserabspaltung die entsprechenden 1-Olefine hergestellt.

Im erfindungsgemäßen Verfahren kann die Dehydratisierung in der Gas- oder Flüssig/Gas-Mischphasc durchgeführt werden. Der Verfahrensschritt c) kann kontinuierlich oder diskontinuierlich durchgeführt werden. Der Verfahrensschritt c) kann an suspendierten oder stückigen im Festbett angeordneten Katalysatoren durchgeführt werden. Die Wasserabspaltung wird wegen der einfachen Abtrennung der Reaktionsprodukte aus dem Reaktionsgemisch bevorzugt an festen Katalysatoren im Temperaturbereich von 200 bis 500 °C in der Gas- oder Gas/Flüssig-Mischphase durchgeführt. Besonders bevorzugt wird eine kontinuierliche Dehydratisierung an einem im Festbett angeordneten Katalysator durchgeführt. Als Katalysatoren können Oxide der Erdalkalimetalle, des Aluminiums, Indiums, Galliums, des Siliziums, Scandiums, Yttriums, Lanthans, Titans, Zirkoniums, Thoriums sowie der seltenen Erden verwendet werden. Es können auch Mischoxide und Kombinationen der obigen Oxide eingesetzt werden. Bei einigen Katalysatoren kann durch Zugabe von Alkalioxiden eine bestimmte Acidität eingestellt werden.

Aus der wissenschaftlichen Fachliteratur sind beispielsweise folgende geeignete Katalysatoren bekannt:
NiO/Al₂-O₃; CuO/Al₂O₃; Al₂O₃ (J. Mol. Catal. A. Chem. (1997), 121 (2 - 3), S. 157 - 159);
ZrO₂; sulfatisiertes ZrO₂ (J. Mol. Cat. A. Chem (1997), 118 (1), S. 88 - 89);
Al₂-O₃; Co₂O₃; ThO₂; In₂O₃ (J. Catal. (1988), 110 (2), S. 416 - 418);
HfO₂/ZrO₂ (J. Phys. Chem. (1980), 84 (1), 55 - 56);
Al₂O₃/Na₂O; ThO2 (J. Catal. (1981), 68 (2), S. 383 - 387);
ThO₂ (J. Org. Chem. (1967), 32 (11), 3386 - 3389);
La₂O₃ (Z. Phys. Chem.(1985), 144, S. 157 - 163);
Ga₂O₃ (J. Org. Chem. (1977), 44 (13), S. 2142 - 2145);
ThO₂; Al₂O₃ (J. Org. Chem. (1972), 37 (8), S. 1240 - 1244);

Vorzugsweise erfolgt die Auswahl der Katalysatoren und der Reaktionsbedingungen so, dass die Bildung von Nebenprodukten, wie beispielsweise von Ethern sowie die Isomerisierung der gebildeten 1-Olefme zu Olefinen mit innenständigen Doppelbindungen weitgehend vermieden wird. Für die Herstellung der 1-Olefine aus den primären Alkoholen durch Wasserabspaltung im Verfahrensschritt c) werden im erfindungsgemäßen Verfahren deshalb bevorzugt basische oder stark basische Katalysatoren eingesetzt. Die eingesetzten Katalysatoren können als Hauptkomponenten Aluminiumoxid (Al₂O₃) und/oder Zirkoniumoxid (ZrO₂) sowie Alkalimetall- und/oder Erdalkalioxide aufweisen. Als weitere Komponenten können im Katalysator Titandioxid, Siliziumdioxid und/oder Thoriumoxid mit 0,01 bis 3 Massen-%, bevorzugt 0,5 bis 5 Massen-% enthalten sein

Der Anteil an basischen Metalloxiden (Hydroxide werden in Oxide umgerechnet) im Katalysator beträgt bevorzugt von 0,01 bis 10 Massen-%, besonders bevorzugt von 0,1 bis 5 Massen-%, insbesondere bevorzugt von 0,1 bis 3 Massen-%. Bevorzugte Alkalimetalloxide sind Natrium-und/oder Kaliumoxid. Als Erdalkalimetalloxide werden bevorzugt Magnesium-, Strontium-und/oder Bariumoxid eingesetzt. Besonders bevorzugt erfolgt die Wasserabspaltung im Verfahrensschritt c) an einem festen Katalysator durchgeführt wird, der formal aus Aluminiumoxid und Bariumoxid besteht. Als Katalysator wird im Verfahrensschritt c) ganz besonders bevorzugt ein mit Bariumoxid (BaO) modifiziertes γ-Aluminiumoxid, welches formal aus Bariumoxid und Aluminiumoxid besteht, verwendet.

Bevorzugt werden γ-Aluminiumoxide mit einer BET-Oberfläche von 80 bis 350 m²/g, bevorzugt 120 bis 250 m²/g (bestimmt durch N₂-Absorption gemäß DIN 66131) eingesetzt. Die Katalysatoren werden nach bekannten Methoden hergestellt. Gängige Methoden sind beispielsweise Fällung, Tränkung oder Besprühung eines Al₂O₃-Körpers mit einer entsprechenden Salzlösung und anschließende Calcinierung.

Ebenso vorteilhaft kann es sein, wenn Katalysatoren eingesetzt werden, wie sie in DE 103 59 628 beschrieben werden und die einen Anteil an Zirkoniumdioxid von 80 bis 99 Massenteile, an Yttriumoxid von 0,5 bis 10 Massenteile und an Erdalkali- oder Alkalioxiden von 0,1 bis 3 Massenteilen aufweisen.

Die Katalysatoren werden vorzugsweise in Form von Kugeln, Tabletten, Zylindern, Strangextrudaten oder Ringen eingesetzt.

Bei der kontinuierlichen Wasserabspaltung können unterschiedliche Verfahrensvarianten eingesetzt werden. Der Verfahrensschritt c) kann z. B. adiabatisch, polytrop oder praktisch isotherm, d. h. mit einer Temperaturdifferenz von typischerweise kleiner als 10 °C, durchgeführt werden. Der Verfahrensschritt kann ein- oder mehrstufig durchgeführt werden. Im letzteren Fall können alle Reaktoren, zweckmäßig Rohrreaktoren, adiabatisch oder praktisch isotherm betrieben werden. Ebenfalls ist es möglich, einen oder mehrere Reaktoren adiabatisch und die anderen praktisch isotherm zu betreiben. Bevorzugt wird die Wasserabspaltung im geraden Durchgang betrieben. Sie kann jedoch auch unter Produktrückführung betrieben werden. Bei Betrieb im geraden Durchgang beträgt die spezifische Katalysatorbelastung 0,01 bis 30 bevorzugt von 0,1 bis 10 kg Alkohol je kg Katalysator und je Stunde. Bei der Wasserabspaltung gemäß Verfahrensschritt c) beträgt die Temperatur in der Katalysatorschicht vorzugsweise von 200 bis 450 °C, insbesondere von 250 bis 350 °C. Die Wasserabspaltung (Dehydratisierung) kann unter vermindertem Druck, Überdruck oder bei Normaldruck durchgeführt werden.

Das Edukt des Verfahrensschritts c), also der in Verfahrensschritt b) erhaltene Alkohol, insbesondere 3-Methylbutanol kann in reiner Form oder in Verdünnung in den Dehydratisierungsreaktor gefahren werden. Als Verdünnungsmittel können inerte Gase oder Gasgemische, wie beispielsweise Stickstoff, Wasserstoff, Kohlenmonoxid, Kohlendioxid, Synthesegas, Methan oder Wasserdampf, oder unter Reaktionsbedingungen inerte organische Lösungsmittel, die vom Reaktionsaustrag leicht abgetrennt werden können, eingesetzt werden.

Um eine möglichst hohe Selektivität hin zur 1-Olefin Bildung zu erzielen, hat es sich als vorteilhaft erwiesen, wenn nur ein Teilumsatz des eingesetzten Alkohols angestrebt wird. Bevorzugt wird der Verfahrensschritt c) so ausgeführt, dass der Umsatz im geraden Durchgang 30 bis 90 % beträgt.

Als Produkt der Verfahrensstufe c) wird ein zumindest ein 1-Olefin, insbesondere 3-Methylbut-1-en aufweisendes Gemisch erhalten. Vorzugsweise wird in Verfahrensschritt c) eine 1-Olefine aufweisende Fraktion erhalten, aus der 3-Methylbut-1-en abgetrennt wird. Das Reaktionsgemisch kann, gegebenenfalls nach Wasserabtrennung, destillativ in Ausgangsalkohol, Olefine und Nebenprodukte getrennt werden.

Das Reaktionsgemisch wird bevorzugt in das/die entstandene 1-Olefin(e), den/die primären Alkohol(e) und gegebenenfalls Nebenprodukte, wie beispielsweise Ether oder Carbonylverbindungen, wie z. B. Aldehyde, aufweisende Fraktionen getrennt. Die Trennung kann z. B. durch Destillation erfolgen. Die nach der Trennung erhaltene Olefinfraktion kann optional zu reinem 3-Methylbut-1-en (weniger als 0,1 Massen-% Fremdstoffe) aufgearbeitet werden. Die Alkohol aufweisende Fraktion, die den nicht umgesetzten Alkohol aufweist, wird vorzugsweise in die Dehydratisierung zurückgeführt. Gegebenenfalls bei der Dehydratisierung als Nebenprodukt entstandener Aldehyd kann nach Hydrierung zum entsprechenden Alkohol wiederverwendet werden und bedingt somit keinen Stoffverlust. Die Hydrierung kann in einer gesonderten Hydrienvorrichtung durchgeführt werden. Es kann jedoch vorteilhaft sein, die als Nebenprodukte erhaltenen Aldehyde in die der Dehydratisierung vorgelagerte Hydrierung (Verfahrensschritt b) einzuspeisen. Durch diese Abtrennung der Aldehyde aus dem bei der Dehydratisierung erhaltenen Gemisch, anschließende Hydrierung und Rückführung der erhaltenen Alkohole in die Dehydratisierung können bei der erfindungsgemäßen Dehydratisierung Olefine in einer besonders hohen Selektivität erhalten werden.

Ist der Ausgangsstoff des erfindungsgemäßen Verfahrens, der in Verfahrensschritt a) eingesetzt wird, beispielsweise ein C₄-Kohlenwasserstoffgemisch, das Isobuten und lineare Butene aufweist oder aus diesen besteht, so wird nach Hydroformylierung, Hydrierung und Wasserabspaltung (Verfahrensstufen a) bis c)) ein C₅-Olefingemisch gewonnen, welches die 1-Olefine 3-Methylbut-1-en, 1-Penten und gegebenenfalls 2-Methylbut-1-en und 2-Methylbut-2-ene aufweisen kann. 1-Penten hat bei Normaldruck einen Siedepunkt von 30 °C, 2-Methylbut-1-en einen von 31,2 °C, 2-Methylbut-2-en einen von 38 °C und 3-Methylbut-1-en einen von 20,1 °C. Auf Grund der deutlich unterschiedlichen Siedepunkte kann 3-Methylbut-1-en nach dem Verfahrensschritt c) leicht von den anderen Isomeren, die in der erhaltenen 1-Olefinfraktion vorliegen, destillativ abgetrennt werden.

Das nach dem erfindungsgemäßen Verfahren gewonnene 3-Methylbut-1-en kann als Monomer oder Comonomer zur Herstellung von Oligomeren oder Polymeren eingesetzt werden. Es kann außerdem als Ausgangsverbindung für die Herstellung von Epoxiden, Ketonen, Aldehyden, Alkoholen und Carbonsäuren verwendet werden. Weiterhin kann es als Alkylierungsmittel oder als Komponente in Enreaktionen verwendet werden.

3-Methylbut-1-en, welches insbesondere nach dem erfindungsgemäßen Verfahren erhalten werden kann, enthält vorzugsweise kleiner-gleich 10 Massen-%, bevorzugt kleiner gleich 1 Massen-% und besonders bevorzugt von 0,001 bis 1 Massen-% 2-Methylbut-1-en, 2.2-Dimethylprop-1-en und/oder 3-Methylbut-2-en. Ein bevorzugtes Gemisch enthält 3-Methylbut-1-en und 2-Methylbut-1-en, 2,2-Dimethylprop-1-en und/oder 3-Methylbut-2-en, wobei der Massenanteil von 3-Methylbut-1-en zumindest 90 Massen-% beträgt und der Massenanteil an 2-Methylbut-1-en, 2,2-Dimethylprop-1-en und/oder 3-Methylbut-2-en weniger als 10 Massen-% beträgt. Vorzugsweise weist das Gemisch zumindest 99 Massen-% und besonders bevorzugt von 99,000 bis 99,999 Massen-% 3-Methylbut-1-en und bevorzugt kleiner gleich 1 Massen-% und besonders bevorzugt von 0,001 bis 1 Massen-% 2-Methylbut-1-en, 2,2-Dimethylprop-1-en und/oder 3-Methylbut-2-en auf, wobei sich die Anteile zu 100 % addieren. 3-methylbut-1-en bzw. das Gemisch kann als Monomer oder Comonomer bei der Polymerisation verwendet werden. Insbesondere kann 3-Methylbut-1-en bzw. das Gemisch als Comonomer oder

Blockcomonomer bei der Polymerisierung/Copolymerisation von Ethylen oder Propylen verwendet werden. Durch diese Verwendung des 3-Methylbut-1-en bzw. des Gemischs können die entsprechenden Polymerisate, also Homo-, Co- und Blockcopolymerisate erhalten werden.

Die folgenden Beispiele sollen das erfindungsgemäße Verfahren erläutern, ohne die Anwendungsbreite einzuschränken, die sich aus der Beschreibung und den Patentansprüchen ergibt.

### Beispiel 1 (Hydroformylierung)

Der Versuch wurde in einer Versuchsanlage, bestehend aus einem Blasensäulenreaktor, einem Dünnschichtverdampfer und einer Destillationseinrichtung durchgeführt. Das Isobuten wurde unten, zusammen mit einem Überschuss an Synthesegas und einem, den Katalysator enthaltenden hochsiedenden Lösungsmittel, in die Blasensäule eingebracht. Am Kopf des Reaktors wurde nicht umgesetztes Synthesegas abgetrennt. Die flüssigen Anteile (Restolefin, Aldehyde, Nebenprodukte, hochsiedendes Lösungsmittel, Katalysator) wurden dem Dünnschichtverdampfer zugeleitet, der unter vermindertem Druck betrieben wurde, sodass hier der gebildete Aldehyd zusammen mit den nicht umgesetzten Olefinen von den höhersiedenden Komponenten, in denen der Katalysator gelöst war, getrennt wurde. Als hochsiedendes Lösungsmittel wurde Dioctylphthalat eingesetzt, das mit 20 % Gewichtsanteil im Reaktor vorlag, weil beim Anfahren des Versuches kein Hochsieder aus dem Prozess vorlag und sich während der Versuchsdauer auch nur wenig bilden würde. Die Rhodiumkonzentration im Reaktor betrug 30 Massen-ppm Rhodium, als Ligand wurde Tris(2.4-ditert.-butylphenyl)phosphit zugesetzt, das molare P/Rh-Verhältnis betrug 20/l. Die Blasensäule wurde von außen über einen Doppelmantel auf konstant 115 °C temperiert, der Betriebsdruck lag bei 5,0 MPa Synthesegas.

Bei den oben angegebenen Reaktionsbedingungen wurde ein Olefinzulauf von kg/h Isobuten eingestellt. Der Blasensäulenreaktor hatte ein Volumen von 2,1 Litern.

Die Bilanzierung der Stoffströme ergab für Isobuten und Folgeprodukte folgende Produktverteilung in Massen-%:

| | |
|---|---|
| Isobuten | 8,2 |
| Pivalinaldehyd | 0,1 |
| 3-Methylbutanal | 90,8 |
| 3-Methylbutanol | 0,3 |
| Hochsieder | 0,6 |

Der Umsatz von Isobuten betrug 92 % bei einer Selektivität zu 3-Methylbutanal bezogen auf Isobuten von 99 %.

### Beispiel 2: Hydrierung von 3-Methylbutanal

Vom Produkt aus Beispiel 1 wurde das nicht umgesetzte Isobuten abdestilliert. Der verbleibende Rest wurde in Reaktionsprodukte und Dioctylphthalat mit geringen Mengen an Hochsiedern fraktioniert. Das Destillat wies folgende Zusammensetzung auf:

| | |
|---|---|
| Pivalinaldehyd | 0,1 |
| 3-Methylbutanal | 99,1 |
| 3-Methylbutanol | 0,3 |
| Hochsieder | 0,5 |

1 kg des Destillats wurden mit 12 g Wasser versetzt und in einem 2 1-Rührautoklaven bei 180 °C an 100 g eines Cu/Cr/Ni-Katalysators auf Al₂O₃-Träger hydriert. Der Katalysator enthielt bezogen auf den Katalysator 0,3 Massen-% Cu, 4,5 Massen-% Ni und 0,07 Massen-% Cr und wurde analog zu Beispiel 1 in EP 0 326 674 hergestellt, wobei statt eines SiO₂-Trägers ein Al₂O₃-Träger eingesetzt wurde. Nach 3 Stunden war die Hydrierung beendet. Nach Abkühlen, Entspannen und Abfiltration des Katalysators wurde ein Hydrierprodukt mit folgender Zusammensetzung (wasserfrei gerechnet) erhalten:

| | |
|---|---|
| 2,2-Dimethylpropanol | 0,1 |
| 3-Methylbutanal | 0,1 |
| 3-Methylbutanol | 99,3 |
| Hochsieder | 0,5 |

### Beispiel 3: Herstellung eines Dehydratisierungskatalysators

Als Trägermaterial für die Herstellung eines Dehydratisierungskatalysators wurde ein saures γ-Aluminiumoxid mit einem Na₂O-Gehalt von kleiner 300 Massen-ppm der Fa. Axens verwendet. Dieses Aluminiumoxid mit einer BET-Oberfläche von 225 m²/g und einem Porenvolumen von 0,68 ml/g lag als Extrudat (Zylinder mit einer Länge von 4 - 6 mm und einem Durchmesser von 1,25 mm) vor. Als Barium-Vorläufer für die basische Modifizierung des Aluminiumoxids mit Bariumoxid (BaO) wurde Bariumnitrat Ba(NO₃)₂ eingesetzt.

Vor der Aufbringung des Bariumsalzes wurde das Aluminiumoxid zuerst bei 90 °C 5 h lang in einem Umlufttrockenschrank getrocknet. 200 g des getrockneten Strangextrudats wurden anschließend in einer Rotationstrommel (Drageetrommel) bei Raumtemperatur mit einer Lösung, bestehend aus 130 ml Wasser und 5,19 g Bariumnitrat, mit Hilfe einer Sprühdüse imprägniert.

Nach der Imprägnierung wurde das mit dem Bariumsalz beladene Extrudat zuerst bei 110 °C 5 h lang, in einem Umlufttrockenschrank getrocknet. Die anschließende Calcinierung, bei der das Bariumsalz zu Bariumoxid bzw. zu einer Barium/Aluminium/Sauerstoffverbindung umgewandelt wird, erfolgte in einem Wirbelbettreaktor im Luftstrom für 10 h bei 450 °C. Der fertige Katalysator enthielt 1,5 Massen-% Bariumverbindungen, berechnet als Bariumoxid.

### Beispiel 4: Herstellung von 3-Methylbut-1-en aus 3-Methylbutan-1-ol

Das in Beispiel 2 erhaltene 3-Methylbutan-1-ol wurde in einem elektrisch beheizten Durchfluss-Festbettreaktor am nach Beispiel 3 hergestellten Katalysator umgesetzt. Vor dem Eintritt in den Reaktor wurde das flüssige Edukt in einem vorgeschalteten Verdampfer bei 220 °C verdampft. Bei einer Reaktionstemperatur von 340 °C wurden stündlich 24 g 3-Methylbutan-1-ol durch 15,1 g Katalysator in der Gasphase, entsprechend einem WHSV-Wert von 1,59 h⁻¹, durchgeleitet. Der Reaktionsdruck betrug 0.15 MPa. Das gasförmige Produkt wurde in einem Kühler abgekühlt und in einer Glasvorlage gesammelt. Das Produkt hatte wasserfrei gerechnet folgende Zusammensetzung:

| Komponente | Gehalt |
|---|---|
| | [Massen-%] |
| 3-Methyl-Buten-1 | 94,4900 |
| 3-Methyl-Buten-2 | 3,2157 |
| 2-Methyl-Buten-1 | 0,6985 |
| Di-(3-Methylbutyl)-Ether | 0,1995 |
| 3-Methyl-Butanol-1 | 0,9105 |
| Hochsieder | 0,4858 |

### Beispiel 5: Reinigung von 3-Methylbut-1-en

10,8 kg des in Beispiel 4 erhaltenen Produktes wurden in einer fraktionierten Destillation in einer Laborapparatur, wie im Folgenden beschrieben, destillativ getrennt. Die Normalsiedepunkte der beteiligten Komponenten sind in der folgenden Tabelle aufgeführt:

| | Normal-Siedepunkt |
|---|---|
| Komponente | [°C] |
| 3-Methyl-Buten-1 | 20,0 |
| 3-Methyl-Buten-2 | 38,5 |
| 2-Methyl-Buten-1 | 31,2 |
| Di-(3-Methylbutyl)-Ether | 130,9 |
| 3-Methyl-Butanol-1 | 173,3 |
| Hochsieder | > 180 |

Wie an Hand der angegebenen Normal-Siedepunkte zu erkennen ist, hat das Gemisch einen relativ breiten Siedebereich. Azeotrope werden nicht gebildet, so dass durch einfache Batch-Destillation eine Aufkonzentrierung von 3-Methyl-Buten-1 möglich ist.

Dazu wurde eine Glaskolonne, bestehend aus einer Destillationsblase mit innen liegendem Verdampfer, 3 Schüssen und Kopfkondensator aufgebaut. Die Kolonnenschüsse hatten einen

Durchmesser von 80 mm und waren mit jeweils 1 m Gewebepackung, Typ Sulzer DX^{®}, bestückt. Laut Herstellerangabe hat dieser Packungstyp ca. 20 theoretische Trennstufen pro Meter Packung, so dass insgesamt ca. 60 theoretische Trennstufen für die Destillation zur Verfügung standen. Für die Kühlung des Kopfkondensators wurde Kaltwasser (Vorlauf ca. 10 °C), für die Sumpfbeheizung (Wärmeträgeröl) verwendet.

Der Sumpf wurde mit 10,8 kg Produkt gefüllt. Anschließend wurde die Kolonne evakuiert und aufgeheizt, wobei bei unendlichem Rücklauf ohne Produktabnahme gefahren wurde. Während der Destillation wurde die Kolonne bei einem Kopfdruck von 0,0980 MPa(abs) und einem Rücklaufverhältnis von 1,6 bis 1,9 betrieben. Dabei ergaben sich Sumpftemperaturen von ca. 39 °C und Kopftemperaturen von ca. 19 °C.

Durch die so beschriebene Betriebsweise der Kolonne konnten aus dem Reaktoraustrag 9,8 kg Produkt mit der folgenden Zusammensetzung generiert werden:

| | Gehalt |
|---|---|
| Komponente | [Massen-%] |
| 3-Methyl-Buten-1 | 99,8051 |
| 3-Methyl-Buten-2 | 0,0001 |
| 2-Methyl-Buten-1 | 0,1948 |
| Di-(3-methylbutyl)-Ether | |
| 3-Methyl-Butanol-1 | |
| Hochsieder | |

## Patentansprüche

1. Verfahren zur Herstellung von 3-Methylbut-1-en aus einem Isobuten aufweisenden Kohlenwasserstoffstrom I,
**dadurch gekennzeichnet, dass**
a) ein Kohlenwasserstoffstrom II, der zumindest 70 Massen-% Isobuten in Bezug auf die im Kohlenwasserstoffstrom enthaltenen Olefine aufweist und der aus dem Kohlenwasserstoffstrom I erhalten wurde oder mit diesem identisch ist, einem Verfahrensschritt der Hydroformylierung zugeführt wird, in der Isobuten in Gegenwart eines Rhodium-Katalysators hydroformyliert wird,
b) der in Schritt a) aus der Hydroformylierung von Isobuten erhaltene Aldehyd zu dem entsprechenden Alkohol hydriert wird,
c) aus dem in Verfahrensschritt b) erhaltenen zumindest einem Alkohol durch Wasserabspaltung 3-Methylbut-1-en hergestellt wird.

2. Verfahren nach Anspruch 1,
**dadurch gekennzeichnet,**
**dass** als Kohlenwasserstoffstrom I oder II ein Gemisch eingesetzt wird, welches Olefine mit 3 bis 5 Kohlenstoffatomen aufweist.

3. Verfahren nach Anspruch 1 oder 2,
**dadurch gekennzeichnet,**
**dass** als Kohlenwasserstoffstrom I oder II ein Gemisch eingesetzt wird, das Isobuten und lineare Butene aufweist oder aus diesen besteht.

4. Verfahren nach zumindest einem der Ansprüche 1 bis 3,
**dadurch gekennzeichnet,**
**dass** als Kohlenwasserstoffstrom I oder II ein C₄-Schnitt, ausgewählt aus Raffinat I, selektiv hydriertem Crack-C₄, C₄-Schnitten aus FCC-Anlagen oder C₄-Olefinen, hergestellt durch Fischer-Tropsch-Synthese, eingesetzt wird.

5. Verfahren nach zumindest einem der Ansprüche 1 bis 4,
**dadurch gekennzeichnet,**
**dass** als Kohlenwasserstoffstrom I technische C₄-Schnitte eingesetzt werden, die einen Isobuten-Gehalt von größer 3 Massen-% aufweisen.

6. Verfahren nach zumindest einem der Ansprüche 1 bis 5,
**dadurch gekennzeichnet,**
**dass** in Verfahrensschritt a) ein Kohlenwasserstoffstrom II eingesetzt wird, der zumindest 85 Massen-% Isobuten bezogen auf die enthaltenen Olefine aufweist.

7. Verfahren nach Anspruch 6,
**dadurch gekennzeichnet,**
**dass** in Verfahrensschritt a) ein Kohlenwasserstoffstrom eingesetzt wird, der zumindest 95 Massen-% Isobuten bezogen auf die Olefine aufweist.

8. Verfahren nach zumindest einem der Ansprüche 1 bis 7,
**dadurch gekennzeichnet,**
**dass** vor dem Verfahrensschritt a) eine Verfahrensstufe 1) durchgeführt wird, durch den aus dem Isobuten aufweisenden Kohlenwasserstoffstrom I ein Kohlenwasserstoffstrom II mit einer höheren Konzentration an Isobuten erhalten wird, der dem Verfahrensschritt a) zugeführt wird.

9. Verfahren nach Anspruch 8,
**dadurch gekennzeichnet,**
**dass** eine Verfahrensstufe 1) durchgeführt wird, die die Verfahrensschritte
1a) Umsetzen von im Isobuten aufweisenden Kohlenwasserstoffstrom I enthaltenem Isobuten mit einer Verbindung V, ausgewählt aus Wasser oder Alkohol, um ein Produkt P zu erhalten,
1b) Abtrennen des Produktes P vom Kohlenwasserstoffstrom I,
1c) Spaltung des Produktes P in Isobuten und Verbindung V und
1d) Abtrennen des Isobutens von der Verbindung V unter Erhalt eines Kohlenwasserstoffstroms II.

10. Verfahren nach Anspruch 9,
**dadurch gekennzeichnet,**
**dass** die in Verfahrensschritt 1a) eingesetzte Verbindung ausgewählt ist aus Wasser oder Alkohol.

11. Verfahren nach Anspruch 9 oder 10,
**dadurch gekennzeichnet,**
**dass** in Verfahrensschritt 1a) und/oder 1c) eine Reaktivdestillationskolonne eingesetzt wird.

12. Verfahren nach zumindest einem der Ansprüche 1 bis 11,
**dadurch gekennzeichnet,**
**dass** in Verfahrensschritt a) ein Rhodium-Komplexkatalysator eingesetzt wird, der phosphor-organische Liganden aufweist.

13. Verfahren nach zumindest einem der Ansprüche 1 bis 12,
**dadurch gekennzeichnet,**
**dass** im Verfahrensschritt b) als Katalysator ein Nickel-, Kupfer-, Kupfer/Nickel-, Kupfer/Chrom-, Kupfer/Chrom/Nickel-, Zink/Chrom-, Nickel/Molybdän-Katalysator eingesetzt wird.

14. Verfahren nach zumindest einem der Ansprüche 1 bis 13,
**dadurch gekennzeichnet,**
**dass** im Verfahrensschritt c) die Wasserabspaltung (kontinuierlich) an einem festen Katalysator durchgeführt wird, der formal aus Aluminiumoxid und Bariumoxid besteht.

15. Verfahren nach zumindest einem der Ansprüche 6 bis 8,
**dadurch gekennzeichnet,**
**dass** in Verfahrensschritt c) eine 1-Olefine aufweisende Fraktion erhalten wird, aus der 3-Methylbut-1-en abgetrennt wird.

## Claims

1. Process for preparing 3-methylbut-1-ene from a hydrocarbon stream I comprising isobutene, **characterized in that**
a) a hydrocarbon stream II which comprises at least 70% by mass of isobutene in relation to the olefins present in the hydrocarbon stream and which has been obtained from hydrocarbon stream I or is identical to it is fed to a process step for hydroformylation in which isobutene is hydroformylated in the presence of a rhodium catalyst,
b) the aldehyde obtained in step a) from the hydroformylation of isobutene is hydrogenated to the corresponding alcohol,
c) 3-methylbut-1-ene is prepared by water elimination from the at least one alcohol obtained in process step b).

2. Process according to Claim 1,
**characterized in that**
the hydrocarbon stream I or II used is a mixture which comprises olefins having 3 to 5 carbon atoms.

3. Process according to Claim 1 or 2,
**characterized in that**
the hydrocarbon stream I or II used is a mixture which comprises isobutene and linear butenes or consists thereof.

4. Process according to at least one of Claims 1 to 3,
**characterized in that**
the hydrocarbon stream I or II used is a C₄ cut selected from raffinate I, selectively hydrogenated crack-C₄, C₄ cuts from FCC plants, or C₄ olefins prepared by Fischer-Tropsch synthesis.

5. Process according to at least one of Claims 1 to 4,
**characterized in that**
the hydrocarbon stream I used is technical C₄ cuts which have an isobutene content of greater than 3% by mass.

6. Process according to at least one of Claims 1 to 5,
**characterized in that**
a hydrocarbon stream II which comprises at least 85% by mass of isobutene based on the olefins present is used in process step a).

7. Process according to Claim 6,
**characterized in that**
a hydrocarbon stream which comprises at least 95% by mass of isobutene based on the olefins is used in process step a).

8. Process according to at least one of Claims 1 to 7,
**characterized in that**
before process step a), a process stage 1) is performed, by which a hydrocarbon stream II having a higher concentration of isobutene which is fed to process step a) is obtained from the hydrocarbon stream I comprising isobutene.

9. Process according to Claim 8,
**characterized in that**
a process stage 1) is performed which comprises the process steps of
1a) reacting isobutene present in the hydrocarbon stream I comprising isobutene with a compound V selected from water or alcohol to obtain a product P,
1b) removing product P from the hydrocarbon stream I,
1c) cleaving product P into isobutene and compound V and
1d) removing the isobutene from compound V to obtain a hydrocarbon stream II.

10. Process according to Claim 9,
**characterized in that**
the compound used in process step 1a) is selected from water or alcohol.

11. Process according to Claim 9 or 10,
**characterized in that**
a reactive distillation column is used in process step 1a) and/or 1c).

12. Process according to at least one of Claims 1 to 11,
**characterized in that**
a rhodium complex catalyst which has organophosphorus ligands is used in process step a).

13. Process according to at least one of Claims 1 to 12,
**characterized in that**
the catalyst used in process step b) is a nickel, copper, copper/nickel, copper/chromium, copper/chromium/nickel, zinc/chromium, nickel/molybdenum catalyst.

14. Process according to at least one of Claims 1 to 13,
**characterized in that**
the water elimination in process step c) is performed (continuously) over a solid catalyst which consists, in a formal sense, of aluminium oxide and barium oxide.

15. Process according to at least one of Claims 6 to 8,
**characterized in that**
a fraction comprising 1-olefins is obtained in process step c), from which 3-methylbut-1-ene is removed.

## Revendications

1. Procédé de fabrication de 3-méthylbut-1-ène à partir d'un courant d'hydrocarbures I comprenant de l'isobutène, **caractérisé en ce que**
a) un courant d'hydrocarbures II, qui comprend au moins 70 % en masse d'isobutène par rapport aux oléfines contenues dans le courant d'hydrocarbures et qui a été obtenu à partir du courant d'hydrocarbures I ou est identique à celui-ci, est introduit dans une étape de procédé d'hydroformylation, lors de laquelle l'isobutène est hydroformylé en présence d'un catalyseur de rhodium,
b) l'aldéhyde obtenu à l'étape a) par l'hydroformylation de l'isobutène est hydrogéné en l'alcool correspondant,
c) du 3-méthylbut-1-ène est fabriqué à partir du ou des alcools obtenus à l'étape de procédé b).

2. Procédé selon la revendication 1, **caractérisé en ce qu'**un mélange qui comprend des oléfines de 3 à 5 atomes de carbone est utilisé en tant que courant d'hydrocarbures I ou II.

3. Procédé selon la revendication 1 ou 2, **caractérisé en ce qu'**un mélange qui comprend de l'isobutène et des butènes linéaires ou qui est constitué de ceux-ci est utilisé en tant que courant d'hydrocarbures I ou II.

4. Procédé selon au moins l'une quelconque des revendications 1 à 3, **caractérisé en ce qu'**une coupe en C₄ choisie par le raffinat I, une coupe de craquage en C₄ hydrogénée sélectivement, les coupes en C₄ d'unités FCC ou les oléfines en C₄ fabriquées par synthèse de Fischer-Tropsch est utilisée en tant que courant d'hydrocarbures I ou II.

5. Procédé selon au moins l'une quelconque des revendications 1 à 4, **caractérisé en ce que** des coupes en C₄ techniques qui présentent une teneur en isobutène supérieure à 3 % en masse sont utilisées en tant que courant d'hydrocarbures I.

6. Procédé selon au moins l'une quelconque des revendications 1 à 5, **caractérisé en ce qu'**un courant d'hydrocarbures II qui comprend au moins 85 % en masse d'isobutène par rapport aux oléfines contenues est utilisé à l'étape de procédé a).

7. Procédé selon la revendication 6, **caractérisé en ce qu'**un courant d'hydrocarbures qui comprend au moins 95 % en masse d'isobutène par rapport aux oléfines est utilisé à l'étape de procédé a).

8. Procédé selon au moins l'une quelconque des revendications 1 à 7, **caractérisé en ce qu'**une étape de procédé 1) est réalisée avant l'étape de procédé a), par laquelle un courant d'hydrocarbures II ayant une concentration en isobutène plus élevée est obtenu à partir du courant d'hydrocarbures I comprenant de l'isobutène, qui est introduit dans l'étape de procédé a).

9. Procédé selon la revendication 8, **caractérisé en ce qu'**une étape de procédé 1) est réalisée, qui comprend les étapes de procédé suivantes :
1a) la réaction de l'isobutène contenu dans le courant d'hydrocarbures I comprenant de l'isobutène avec un composé V choisi parmi l'eau ou un alcool, afin d'obtenir un produit P,
1b) la séparation du produit P du courant d'hydrocarbures I,
1c) le clivage du produit P en isobutène et composé V, et
1d) la séparation de l'isobutène du composé V pour obtenir un courant d'hydrocarbures II.

10. Procédé selon la revendication 9, **caractérisé en ce que** le composé utilisé à l'étape de procédé 1a) est choisi parmi l'eau ou un alcool.

11. Procédé selon la revendication 9 ou 10, **caractérisé en ce qu'**une colonne de distillation réactive est utilisée à l'étape de procédé 1a) et/ou 1c).

12. Procédé selon au moins l'une quelconque des revendications 1 à 11, **caractérisé en ce qu'**un catalyseur de complexe de rhodium qui comprend des ligands organiques de phosphore est utilisé à l'étape de procédé a).

13. Procédé selon au moins l'une quelconque des revendications 1 à 12, **caractérisé en ce qu'**un catalyseur de nickel, de cuivre, de cuivre/nickel, de cuivre/chrome, de cuivre/chrome/nickel, de zinc/chrome, de nickel/molybdène est utilisé à l'étape de procédé b) en tant que catalyseur.

14. Procédé selon au moins l'une quelconque des revendications 1 à 13, **caractérisé en ce que** le clivage d'eau à l'étape de procédé c) est réalisé (en continu) sur un catalyseur solide qui est constitué formellement d'oxyde d'aluminium et d'oxyde de baryum.

15. Procédé selon au moins l'une quelconque des revendications 6 à 8, **caractérisé en ce qu'**une fraction comprenant des 1-oléfines est obtenue à l'étape de procédé c), à partir de laquelle le 3-méthylbut-1-ène est séparé.
